# EUROPEAN PATENT SPECIFICATION

(11) **EP 3 531 128 B1**
(45) Date of publication and mention of the grant of the patent: **30.06.2021**
(21) Application number: 19158833.4
(22) Date of filing: 22.02.2019
(51) Int. Cl.: G01N 33/52, G01N 33/543

(54) **INTEGRATED PLATFORM FOR SINGLE CELL ANALYSIS**
INTEGRIERTE PLATTFORM ZUR EINZELZELLANALYSE
PLATE-FORME INTÉGRÉE POUR ANALYSE D'UNE CELLULE UNIQUE

(30) Priority: 22.02.2018 DE 102018104022
(43) Date of publication of application: 28.08.2019
(73) Proprietor: Albert-Ludwigs-Universität Freiburg, 79098 Freiburg (DE)
(72) Inventor: ZINGGELER, Marc, 4052 Basel (CH); RÜHE, Jürgen, 79356 Eichstetten (DE); BRANDSTETTER, Thomas, 79110 Freiburg (DE)
(74) Representative: Hertin und Partner Rechts- und Patentanwälte PartG mbB

(56) References cited:
- US-A1- 2016 045 884
- JING WU ET AL: "Microfluidic technologies in cell isolation and analysis for biomedical applications", ANALYST, vol. 142, no. 3, 1 January 2017 (2017-01-01), pages 421-441, XP055596310, UK ISSN: 0003-2654, DOI: 10.1039/C6AN01939K

## Description

### Introduction

The invention relates to a method for analyzing one or more single cells of interest, comprising a) provision of (i) a porous membrane comprising at least one pore, wherein the pore is adjusted to trap a single cell of interest when the cell is applied to the membrane in a liquid medium, and to allow smaller or more flexible cells of a cell suspension to pass through the pore (ii) a well plate comprising at least one well, wherein the well diameter is larger than the pore diameter and the at least one well contains reagents for analyzing at least one component of the cells of interest, wherein the reagents can be provided in liquid or dry form, (iii) means for assembling the porous membrane and the well plate in an orientation to position the at least one pore opposite of the at least one well, wherein the means for assembling are part of the porous membrane and/or the well plate, (iv) a cover for sealing the at least one well with the at least one pore localized opposite of the at least one well after assembling the porous membrane and the well plate; and b) filtering a liquid cell suspension comprising cells of interest through the porous membrane; c) assembling the porous membrane and the well plate in an orientation to position the at least one pore opposite of the at least one well; d) sealing the at least one well and the at least one pore localized opposite of the well with the cover, and e) analysis of at least one cellular component of a retained single cell in the at least one sealed well.

### Background of the invention

Cancer is a major cause of death and morbidity worldwide (1). In Europe alone, there were an estimated 3.45 million new cases of cancer and 1.75 million deaths from cancer in 2012 (2). Including the most common types of cancer like breast, colorectal, prostate and lung cancer, carcinomas represent by far the most abundant class of tumors. Carcinomas are solid tumors derived from epithelial tissue and most deaths from this class of tumors are caused by the haematogenous spread of cancer cells from the primary tumor into distant organs and their subsequent growth to metastases (3, 4). During this complex process circulating tumor cells (CTCs) were found to play a key role and their reliable detection and characterization could enable new and effective strategies for cancer diagnosis, monitoring and treatment (4-7). However, the analysis of CTCs still represents an analytical grand challenge due to their ultra-low abundance of a few cells per mL of blood covered by billions of blood cells and their complex inter-cell heterogeneity (7-9). This heterogeneity arises from the genetic instability of tumor cells and is suspected to drive metastatic disease, drug resistance and disease progression by an evolutionary process known as clonal evolution (10-12). Resolving this heterogeneity both in the genome and transcriptome of CTCs holds great promise for the targeted treatment of cancer and has shifted the interest from bulk analysis of CTCs towards single-cell methods (5, 11, 13-15).

The development of efficient tools for the isolation of CTCs from whole blood has been an active field of investigation in the past few years and the developed methods typically exploit either biological or physical properties of CTCs for isolation. Methods based on biological separation criteria mostly use the epithelial cell adhesion molecule (EpCAM) typically expressed on the surface of CTCs of epithelial origin as marker (16).

However, there is considerable doubt rising whether a pure EpCAM based isolation method is able to capture all types of CTCs. This is due to the mentioned heterogeneity of CTCs, which is increasingly acknowledged. Especially a process known as epithelial-mesenchymal transition (EMT), which is suspected to provide CTCs with increased invasiveness, was found to cause down-regulation of epithelial markers including EpCAM (17).

Because of this, there has been an increased interest in marker independent separation methods relying on less specific physical separation criteria (reviewed in 18, 19). In this domain most abundantly size/deformability based methods can be found. The simplest devices enabling the highest throughput are based on classical dead-end microfiltration. These methods rely on the larger size and lower deformability of CTCs compared to blood cells for isolation. However, due to significant size overlaps between CTCs and some WBCs, these methods generally suffer from low selectivity, which complicates the downstream analysis of the captured cells (20).

Another issue with existing isolation methods for CTCs is that there exists no truly integrated platform that allows for direct single-cell analysis after CTC capture. First single-cell studies, which were performed on CTCs therefore used complex workflows to combine the enrichment and single-cell analysis of CTCs (reviewed in 10, 13, 21-29). As an example, in the pioneering work by Lohr et al (30) epithelial CTCs were first isolated using anti-EpCAM labeled magnetic beads. Secondly, the isolated CTCs were transferred to a micro-well array for single-cell separation and identification via ICC. Identified CTCs were then transferred to individual wells on a PCR plate by a robotic micromanipulator. Whole-genome amplification (WGA) was then performed by multiple displacement amplification (MDA) followed by whole-genome sequencing using the Illumina MiSeq platform.

Another system offered by the company VyCap combines cell enrichment by filtration and single-cell isolation. For this purpose, a microtiter plate is used which has holes (pores) on the bottom. After enrichment, the captured cells are selectively punched out from the plate into individual wells of a standard well plate for single-cell analysis. However, for this the user requires a sophisticated puncher system (micromanipulator). Alternatively, the reagents could in principle be added to the cells in the wells on the enrichment plate. However, this addition would have to be accomplished by the user and is considered technically challenging at this stage. This procedure is therefore also not part of the offered product. Further, the prefabricated porous microtiter plate is considered disadvantageous due to the high production costs, strongly limited combinatorial freedom of the user (no possibility to combine membrane and well plate individually) and no clean single cell separation (several cells per well) for concentrated cell suspension (such as whole blood).

Furthermore, the company Fluidigm offers a product with an effective combination of single-cell isolation and analysis. However, it requires a target cell suspension as a feed and contains no means for CTC-enrichment. To use this system the user therefore requires an additional CTC-enrichment technology.

The drawbacks of such workflows are the multiple cell transfer steps involved, which could introduce cell-stress and bear high risks for cell losses and contamination. Additionally, the throughputs, which can be obtained with such workflows are strongly limited.

Nagai et al (Moeto Nagai, Kiyotaka Oohara, Keita Kato, Takahiro Kawashima, Takayuki Shibata; Biomed Microdevices (2015) 17: 41; DOT 10.1007/s10544-015-9943-z) have developed an array of hollow probes using microelectromechanical systems fabrication technology and demonstrated the parallel manipulation of single cells that can be captured on the probe by applying negative pressure to the probes. Trapped cells can be transported to a cell culture chip.

US 2011/0233148 A1 discloses a filter plate system that can be used for in-vitro culture of cells or cellular aggregates, and allows for relatively easy and efficient changes of culture media. The disclosed system can comprise a reservoir plate with at least one well, a strainer plate with at least one strainer well comprising at least one mesh wall portion, and a lid. The system is used to filter cellular aggregates from a liquid medium and separate these from single cells, cellular debris and other medium components that can migrate through the mesh wall portion, which preferably has a pore size of 20 to 100 micrometers. This system however is not suitable for isolating single cells, since these are not retained by the strainer well. Furthermore, a strainer well comprises a mesh well portion with multiple, preferably thousands of pores, which are located in or opposite of a single well of the reservoir plate. Accordingly, the system is not suited for the isolation of single cells, but only for the separation of non-aggregated individual cells from cellular aggregates. Isolation, analysis and comparison of one or more individual single cells by microscopy or with respect to the analysis of cellular components is not possible with this system. It is only possible to microscopically observe the cellular aggregates during the cultivation phase in the filter plate system. However, for analysis of single cells or single cell components the cellular aggregates have to be removed or harvested from the system. Thereafter the cells can be dissociation to single cells and subsequently be analyzed in various ways. However, US 2011/0233148 A1 does not describe a device, system or kit that enables isolation and potentially also observation of the isolated individual cells and subsequent analysis of a cellular component in an integrated way. In contrast, many independent subsequent processes have to be performed and no suitable devices for performing all of the steps are described.

US 2016/0045884 A1 discloses a capture chip for capturing single cells from a suspension comprising a substrate with a plurality of cell-sized dimples for trapping the cells. This substrate basically is a multi-well plate, which can be assembled with a through-hole multi-well chip after single cells have been captured in the wells. Importantly, the dimples or wells of the substrate are closed at the bottom, so that cells cannot transition through the substrate, which critically differentiates such substrates from a porous membrane, such as a filter membrane with defined pore size, since cells of different diameters can be captured leading to a non-cell-type specific isolation of single cells from a suspension. The captured single cells can subsequently be analyzed, for example by PCR or gene expression. However, in the methods and systems disclosed in US 2016/0045884 A1 it is not possible to control the specificity of the cell capture, so that it is not possible to be sure that the correct cell type of interest has been captured and analyzed by a certain well or dimple. Furthermore, since the substrate capture chip is closed at the bottom the system is prone to unspecific cell isolation, leading to isolation and analysis of other cell type from the cell suspension than the cells of interest. Such wrongly isolated cells cannot be removed due to the lack of a cell selective surface coating (e.g. containing an immobilized antibody directed against a target cell specific surface marker), which only retains the cells of interest during a subsequent washing step. Further, wrongly isolated cells cannot be identified during or after performing the method, since the system does not comprise a cellular analysis, for example by examining the isolated cells microscopically.

A need exists in the field of enriching, isolating and analyzing single cells for providing means for isolating specific single cells of interest (e.g. CTCs) with a high specificity from a multitude of background cells (e.g. blood cells) in a simple work flow that allows high throughput analysis while preventing loss of cells or contamination of single cell samples.

### Summary of the invention

In light of the difficulties of the prior art, the technical problem underlying the present invention is the provision of means for isolating specific single cells of interest with a high specificity in a simple work flow that allows high throughput analysis while preventing loss of cells or contamination of single cell samples.

The present invention therefore seeks to provide a method, device, kit and system for isolating and analysing single cells of interest that ensure a high specificity for the cell of interest in an integrated system ensuring high throughput analysis of cells while avoiding excessive handling steps that might lead to loss or contamination of isolated single cell samples.

The solution to the technical problem of the invention is provided in the independent claims. Preferred embodiments of the invention are provided in the dependent claims.

The present invention relates to a method for analyzing one or more single cells of interest, comprising
- provision of
   i. a porous membrane comprising at least one pore, wherein the pore is adjusted to trap a single cell of interest when the cell is applied to the membrane in a liquid medium and to allow smaller or more flexible cells of a cell suspension to pass through the pore J Z
   ii. a well plate comprising at least one well, wherein the well diameter is larger than the pore diameter and the at least one well contains reagents for analyzing at least one component of the cells of interest, wherein the reagents can be provided in liquid or dry form,
   iii. means for assembling the porous membrane and the well plate in an orientation to position the at least one pore opposite of the at least one well, wherein the means for assembling are part of the porous membrane and/or the well plate,
   iv. a cover for sealing the at least one well with the at least one pore localized opposite of the at least one well after assembling the porous membrane and the well plate, and
- filtering a liquid cell suspension comprising cells of interest through the porous membrane,
- assembling the porous membrane and the well plate in an orientation to position the at least one pore opposite of the at least one well,
- sealing the at least one well and the at least one pore localized opposite of the well with the cover,
- analysis of at least one cellular component of a retained single cell in the at least one sealed well.

The method of the present invention combines cell enrichment and cell isolation via means of filtration, preferably affinity filtration, with at least one single cell analysis steps. The method of the present invention allows the isolation and analysis of single cells of interest in an integrated method or system, which minimizes handling steps during isolation and analysis of the cells. The method of the present invention reduces the risk of contamination of single cell samples and the analysis of wrongly isolated cells, which do not have the intended identity of the cells of interest, and therefore provides a reliable, fast and efficient (in terms of time and costs) way of gaining information about cells of a cell-population on the single cells level.

With the method of the present invention it is possible to filter a cell suspension, comprising a complex mixture of different cells, through a porous membrane containing one or more pores, preferably hundreds or thousands of pores, wherein each pore of the membrane can retain a single cell of interest of the cells suspension, which gets mechanically trapped or stuck in the pore or adheres to the pore.

In the context of the present invention, trapping of a cell in a pore of the porous membrane relates to the adherence of a cell to the pore after application of the liquid cell suspension containing the cell of interest to the porous membrane, for example in a filtration step. The pore diameter may be adjusted to prevent a single cell of interest from passing through the pore when the cell is applied to the membrane in a liquid medium. Such trapping or adhering of a cell may be achieved mechanically, when the pore diameter is adjusted to the size and rigidity or flexibility of the cell type of interest, so that is allows the cell to enter into the pore but not to pass through the pore, resulting in the cell being trapped or stuck inside the pore.

Alternatively, the pore may be adjusted to display surface properties that allow specific adherence of the cell of interest, but not of other cell types. In this case, the pore diameter may also be bigger that the diameter of the cell of interest, because the cell does not have to be trapped mechanically but can adhere to the pore due to surface adherence through interaction with the surface of the pore. However, in this case it may be advantageous that the pore diameter is about the same or only slightly bigger than the diameter of a cell of interest to prevent the cell from passing through the pore without getting in touch or in close proximity to the surface of the pore, which can interact with the surface of the cell.

A skilled person is able to adjust the pore to be suitable for trapping a specific cell of interest either by adjusting the diameter of the pore for mechanically trapping the cell of interest (such an adjustment may be based on the known average diameter of a cell of interest, its rigidity and/or its flexibility), by adjusting or modifying the surface properties of the pore surface, for example by coating the pore surface with molecules that specifically interact with the cell of interest. In embodiments, the pore may be adjusted for trapping a cell of interest by a combination of adjusting the pore diameter to the cell type of interest and adjusting the surface properties of the cells to allow adherence of a cell of interest, resulting in a combination of mechanical trapping and trapping through specific surface-surface interaction.

The pore is adjusted to prevent a single cell of interest from passing through the pore, and to allow other cell types of the cell suspension to pass through the pore, because they are smaller or more flexible and do not adhere to the pore, and/or to prevent cell types of the cell suspension from passing through or adhering to the pore, because they are too big or rigid to even enter the pore (preventing them from getting mechanically stuck in the pore) and do not display surface properties that allow adherence to the pore surface through specific interaction.

In case of an affinity filtration, cells of interest also bind to the surface of the pore, while other cells do not bind to the surface of the membrane/pore. Accordingly, after filtering the cell suspension through the porous membrane, single cells of interest are sticking to the pores of the membrane, while the other cells of the cell suspension have either passed through the pores without getting stuck or could not pass through the membrane and also do not stick to the pores.

After filtration of the cell suspension through the porous membrane, the porous membrane can be assembled with a well plate. The porous membrane and the well plate of the present invention have been fabricated in a way, for example by microfabrication, that the positions of the pores on the membrane are known and are preferably arranged in an ordered structure on the membrane, while the wells of the well plate are located at positions corresponding to positions of the pores on the membrane and are preferably arranged in the same ordered structure as the pores on the membrane. Accordingly, the position of each pore on the membrane corresponds to a position of a well on the well plate. Upon assembly of the well plate and the porous membrane, the pores are positioned on top of or opposite of a well of the well plate.

The membrane and/or the well plate of the present invention are equipped with means for assembling the porous membrane and the well plate. These means for assembling are ensuring that upon assembly of the membrane and the plate, each pore is located opposite to or on top of its corresponding well and there is no shift in the positioning of the pores on top of the wells. The wells of the well plate have a diameter, which is larger than the diameter of the pores of the membrane, which ensures that after assembly a single cell of interest, which sits in a pore of the porous membrane and is therefore located opposite of a corresponding well, is located in the well and does not stick out or protrude over the edge of the lateral wall of the well.

As a result of the assembly, an isolated single cell that is located in a pore of the membrane is in contact only with the content of one well of the well plate and is assigned to one specific well of the plate. No individual handling steps with respect to single cells are required to transfer the isolated single cells to a specific well, because by assembling the membrane, comprising multiple isolated and identity-verified single cells of interest, and the well plate, each of the, for example, hundreds or thousands of isolated single cells can be positioned in/on/opposite of an individual well. This very efficient and contamination-minimizing step ensures a high quality of the cellular samples and minimizes the risk of cross-contamination between wells.

In embodiments of the invention the means for assembling are part of the cover for sealing.

Examples of means for assembling would be mechanical structures located on the plate and/or the membrane and/or the cover that allow assembly of the plate and the membrane only in one specific orientation, which ensures positioning of the pores opposite of their corresponding wells.

In embodiments of the invention, the method for analyzing one or more single cells of interest additionally comprises a microscopic analysis of the at least one pore for retained single cells.

This microscopic analysis preferably occurs after filtration of the cell suspension. Microscopic analysis may occur prior to or after assembling the porous membrane and the well plate. In embodiments of the invention the microscopic analysis can be performed before or after sealing the at least one well and the at least one pore localized opposite of the well.

Embodiments of the invention can combine cell enrichment and cell isolation via means of filtration, preferably affinity filtration, with at least two single cell analysis steps, wherein these analysis steps comprise a microscopic analysis of the isolated cell of interest, allowing to identify the isolated cell of interest and to confirm its identity, and an analysis of at least one cellular component of an isolated single cell of interest. Therefore, the method of the present invention allows the isolation and analysis of single cells of interest with confirmed identity in an integrated method or system, which minimizes handling steps during isolation and analysis of the cells. This embodiment of the method of the present invention reduces the risk of contamination of single cell samples and the analysis of wrongly isolated cells, which do not have the intended identity of the cells of interest. This method therefore provides a reliable, fast and efficient (in terms of time and costs) way of gaining information about cells of a cell-population on the single cells level.

After the filtering step the porous membrane with the single cells of interest sticking in the pores can be analyzed microscopically to verify that the cells sticking in the pores of the membrane are indeed cells of interest and no other cells of the complex cell suspension. For this analysis step, any kind of microscopic analysis that allows identification of cells of interest can be used. One preferable example would be immunocytochemical (ICC) analysis using cell-specific antibodies.

Preferably, the whole membrane containing the isolated cells is analyzed in a way so that no individual handling, staining, or manipulation of single cells is required. Accordingly, the whole membrane with all isolated cells can be analyzed in a single analysis procedure, which might involve staining of the cells with specific reagents such as specific antibodies.

Microscopic analysis of the membrane can be performed before or after assembling the porous membrane and the well plate. In general, the method steps of the present invention are not listed in a specific chronological order, which represents a set of sequential events that have to occur in the listed order. Instead, the order of the method steps can vary and can be different from the listed order. Accordingly, the order of the different method steps is not limiting with respect to preferred embodiments of the present invention.

In embodiments of the invention, the method steps are performed in the order of
1. filtering the cell suspension through the porous membrane,
2. assembling the porous membrane and the well plate in an orientation to position the at least one pore opposite of the at least one well,
3. sealing the at least one well and the at least one pore localized opposite of the well with the cover,
4. analysis of at least one cellular component of a retained single cell in the at least one sealed well.

Preferably, the microscopic analysis occurs after filtering the cell suspension and before analysis of the at least one cellular component of a retained cell.

In preferred embodiments of the invention, each well of the well plate with the corresponding pore and potentially a retained single cell of interest is sealed before the at least one cellular component of the retained single cell of interest is analyzed. Before sealing, the well is positioned opposite of the pore so that the single cell is located on top of or inside the well without sticking out over the lateral walls of the well.

Sealing of each well with the corresponding pore and single cell located on top or inside the well can be achieved by placing or pressing a cover on the side of the membrane, which is not facing the well plate after assembly of the well plate and the membrane. The sealing of the wells can be achieved, for example, by mechanical and/or thermic means, for example by pressing the cover onto the membrane, which in turn is pressed onto the well plate, while the cover and/or the plate can be warmed or heated.

Sealing of the individual wells with the corresponding pores and single cells ensures that each sealed well comprises only one pore, which is located on top of the well after assembly of the plate and the membrane. Accordingly, each well also only contains one isolated single cell of interest, which sits in the corresponding pore. By sealing a well comprising its corresponding pore with an isolated single cell, contamination of other, for example neighboring wells is prevented. This is necessary, because the analysis of one or more cellular components of single cells requires closed reaction spaces, which may not be accessed by contaminants.

The analysis of at least one cellular component of an isolated single cell of interest, which is located in a sealed well after assembling the membrane and the plate and sealing with the cover, relates to analysis of any kind of biomolecule comprised by the isolated single cell of interest. For example, the analysis can relate to a whole genome sequencing analysis or an analysis of the specific mRNAs, for example by quantitative RT-PCR, or the whole transcriptome of a cell. The reagents required for such an analysis are already present in the wells of the plate, preferably already in liquid form. Alternatively, the wells can contain the reagents in dry form, which requires a rehydration step, which could be performed before assembly of the plate and the membrane, or after assembly, for example by means of a microfluidic system that allows the provision of liquids to the individual sealed wells.

In embodiments of the invention, the cover plate can contain reagents for analyzing at least one component of the cells of interest. Such reagents contained by the cover plate may be present instead of the reagents or in addition to the reagents contained in the at least one well of the well plate.

It is a particular advantage of the present invention that cellular components of isolated single cells can be analyzed without having to transfer the individual cells by complicated means, such as a micromanipulator, to a different analysis system. Instead, the individualized single cells of interest that are located in the pores of the membrane after filtration can be all transferred in one step to individual wells of the plate, in which the subsequent analysis of cellular components can be performed.

The sealing step of the method of the present invention allows the analysis of cellular components of multiple individual cells in an integrated system that does not require handling or manipulation of individual cells. By filtering the cell suspension through the porous membrane, single cells of interest get separated from each other by sitting as single cells in individual pores of the membrane. The isolated/separated individual cells located in the pores of the membrane may be analyzed microscopically to ensure that the cell in a specific pore of the membrane is an intended cell of interest. By assembling the well plate and the porous membrane with the individual isolated cell of interest by using the means for assembly, which ensures the correct orientation of corresponding wells and pores, the individual cells are placed into individual wells without needing to manipulate individual cells. The sealing with a cover ensures that there is no cross-contamination of components of single cells between the different wells. Accordingly, the present invention enables the analysis of single cells without a need to sort or manipulate or move the single cells individually. This allows the parallel analysis of a high number of single cells in a very efficient way, which is fast and reliable.

A "cell" or a "cell of interest" in the sense of the present invention refers to, without limitation, a biological cell, which might be derived from any kind of organism, comprising unicellular organisms as well as multicellular organisms, such as for example any kind of plant or animal, including mammals, fish, amphibians, reptiles, birds, molluscs, arthropods, annelids, nematodes, flatworms, cnidarians, ctenophores and sponges. Cells of the present invention further comprise prokaryotic cells, bacteria, eukaryotic cells, blood cells, stem cells, immune cells (such as B-cells, dendritic cells, granulocytes, innate lymphoid cells (ILCs), megakaryocytes, monocytes, macrophages, myeloid-derived Suppressor Cells (MDSC), natural killer (NK) cells, platelets, red blood cells (RBCs), T-cells or thymocytes), cancer cells, tumor cells and circulating tumor cells.

The term "cell" as used in the context of the present invention, also reads to the term "biological particle" and specific embodiments of particles described herein. Accordingly, all preferred embodiments of the present invention disclosed herein read on both a method for analysis of single cells of interest as well as on a method for analyzing one or more single biological particles of interest. Furthermore, the preferred embodiments of the methods of the present invention disclosed herein also read on the device, the system and the kit for analyzing single cells of interest of the present invention and are herewith also disclosed in the context of these embodiments of the invention.

The present invention further relates to a method for analyzing one or more single biological particle of interest, comprising
- provision of
   i. a porous membrane comprising at least one pore, wherein the pore is adjusted to trap a biological particle of interest when the particle of interest is applied to the membrane in a liquid medium and to allow smaller or more flexible cells of a cell suspension to pass through the pore J Z
   ii. a well plate comprising at least one well, wherein the well diameter is larger than the pore diameter and the at least one well contains reagents for analyzing at least one component of the cells of interest, wherein the reagents can be provided in liquid or dry form,
   iii. means for assembling the porous membrane and the well plate in an orientation to position the at least one pore opposite of the at least one well, wherein the means for assembling are part of the porous membrane and/or the well plate,
   iv. a cover for sealing the at least one well with the at least one pore localized opposite of the at least one well after assembling the porous membrane and the well plate, and
- filtering a liquid suspension comprising biological particles of interest through the porous membrane,
- optional microscopic analysis of the at least one pore for retained single biological particle,
- assembling the porous membrane and the well plate in an orientation to position the at least one pore opposite of the at least one well,
- sealing the at least one well and the at least one pore localized opposite of the well with the cover,
- analysis of at least one biological component of a retained single biological particle in the at least one sealed well.

The term "biological particle" in the sense of the present invention comprises, without limitation, cells of the present invention, single cell organisms, multicellular microorganisms, parasites, viruses, viral particles, viral capsids, bacteriophages, phages, phage capsids and artificial viral capsids. Particles further comprise any kind of beads or microbeads, which are coupled to biomolecules, such as magnetic beads, streptavidin coated beads, biotin-coated beads, antibody coated beads and bead that are coupled to nucleic acid molecules.

The advantages and embodiments disclosed for the method for analyzing one or more single cells of interest are also disclosed for the embodiments of the invention relating to a method for analyzing one or more single biological particles of interest.

The porous membrane of the present invention is used for filtering the cell suspension comprising the cells of interest. The membrane may be plane or laminar and may have a defined thickness. The shape of the membrane can be adjusted to the specific application, for example to be compatible with the means used for filtering the cell suspension through the porous membrane. The shape of the membrane is not limiting for the scope of the present invention.

### Preferred embodiments of the porous membrane of the invention

The porous membrane may be a microfabricated membrane that comprises at least one pore, preferable multiple pores, preferably 1000 - 1.000.000 pores.

Furthermore, the pores preferably have all an identical or almost identical pore diameter, which is specifically adjusted to the size and shape of the cell of interest. Preferably, the pore diameter is such that the diameter of a single cell of interest is slightly bigger than the pore diameter so that the cell of interest cannot pass through the pore during filtration of the cell suspension through the porous membrane, at least not without getting in contact with the boundaries of the pore. In the context of the invention, the pore can be of different geometrical shapes with different shapes of the pore cross section. Preferably, the pore has a round or elliptic cross section and is of conical or cylindrical shape. However, any kind of pore geometry suitable for retaining a single cell of interest in comprised by embodiments of the present invention.

The porous membrane of the present invention preferably comprises a polymer, such as a thermoplastic polymer. Preferred are porous membranes that are polycarbonate filters, silica nitride microsieves, copper or nickel TEM (transmission electron microscopy) grids, membranes comprising polycarbonate, SU-8, parylene, ORMOCER® or silicon.

ORMOCER® is a class of inorganic-organic hybrid polymers. ORMOCER® has properties like transparency, hardness or thermal/chemical stability, which are determined by the inorganic network. By crosslinking it with an organic network it is possible to adapt properties like toughness, to improve the processability or the functionality. By adding functional groups, further properties can be specifically tailored, including elasticity, surface energy or gas permeability. Their unique adaptability is the major advantage of ORMOCER® materials and renders them attractive for the application in the context of the present invention, especially for producing the porous membrane.

In preferred embodiments of the present invention, the membrane is coated with a hydrogel and/or comprises immobilized antibodies.

Such surface modifications of the porous membrane by coating with a hydrogel and/ immobilized antibodies are advantages, because the porous membrane can be modified by such means to become an affinity membrane. The hydrogels and/or antibodies can have certain affinity properties that can enhance specific binding of cells of interest in the pore of the membrane and decrease unspecific binding of unwanted cells or components of the liquid cell suspension. This enables specific retaining of cells of interest in pores of the membrane, even if the cells could pass through the pores of an unmodified membrane. For example, if a cell of interest could pass through a pore of a diameter that is equal or smaller than the diameter of the cell, because the cell is flexible and can adjust its shape to pass through the pore, it might be retained in the pore, if the surface of the membrane or the pore have been modified in a way that leads to a specific binding of the cell of interest.

Hydrogels of the present invention are water-swellable, polymer networks, which can protect surfaces effectively from non-specific adhesion of proteins and cells. When functionalized with specific groups, hydrogel coatings allow to trigger the specific adhesion of cells.

Preferred hydrogels to be used for coating the membranes of the present invention comprise, without limitation, alginate hydrogels, pluronic hydrogels, gelatin hydrogels or chitin hydrogels. Common ingredients of hydrogels include, without limitation, polyvinyl alcohol, sodium polyacrylate, acrylate polymers and copolymers with an abundance of hydrophilic groups. Natural hydrogel materials comprise agarose, methylcellulose, hyaluronan, and other naturally derived polymers.

In a specifically preferred embodiment of the present invention, the substrate can be coated with a hydrogel of polydimethyl acrylamide co methacryloyl benzophenon (PDMAA-MABP), which can contain a photoactive cross-linker benzophenone (BP). The coating can be achieved by dip coating and subsequently illuminated with UV-light (pre-exposure) to initiate both cross-linking and surface-attachment by C-H insertion reactions.

The surface of the porous membrane, which may be coated with a hydrogel already, may be modified by immobilizing or attaching biomolecules with a specific affinity for other biomolecules or cells on the membrane. Preferred modifications of membrane surfaces comprise immobilization of streptavidin, antibodies, aptamers and receptors or receptor ligands, such as, for example, Fc-receptors.

Immobilization of such biomolecules, preferably proteins, with a specific affinity on the surface of a PDMAA-MABP coated membrane may be performed after dip coating and subsequent illumination with UV-light (pre-exposure) to initiate both cross-linking and surface-attachment by C-H insertion reactions by saturating the surface, which may still contain active BP units, with the protein, for example streptavidin, followed by another UV-treatment and a washing step to remove non-bound protein.

If the hydrogel coated surface has been modified by immobilization of streptavidin, the modification process may be completed by binding a target cell specific biotinylated capture molecules, such as an antibodies or an aptamer, to the membrane by simple incubation. This process of surface modification is particularly advantageous, because it yields a homogeneous distribution of stably immobilized proteins and allows to control the degree of functionalization by varying the energy dose of the pre-exposure.

Preferred antibodies or other molecules used for modification of the membrane surface for isolation of specific cells of interest may bind to specific receptors or biomolecules on the surface of the cells of interest.

Preferred immobilized antibodies of the present invention comprise, without limitation, antibodies with an affinity for EpCAM, EGFR, HER2 and MUC-1.

According to a preferred embodiment of the present invention, the pore diameter is in the range of about 10 nm to 1000 µm, preferably 100 nm to 100 µm, more preferably 1 µm to 50 µm, most preferably 5 to 20 µm.

It is a great advantage of the present invention that it is possible to isolate single cells or biological particles of very different sizes from a complex liquid cell suspension, which comprises a variety of biological particles and/or cells. The pore diameter of the membrane can be adjusted to the diameter of the cell or particle of interest. For example, viral particles, viral capsids and artificial viral capsids are usually in the range of 20 to 300 nm, depending on the type of virus or capsid. Most prokaryotes are between 1 µm and 10 µm in size, but their size can vary from 0.2 µm (Mycoplasma genitalium) to 750 µm (Thiomargarita namibiensis), depending on the type. Eukaryotic cells also vary in size but are usually in the range of 2 - 20 µm in diameter, but may be also smaller or lager. For example, megakaryocytes are about 160 µm in diameter and therefore would require larger pore sizes for isolation in the context of the present invention in comparison to for example erythrocytes, which have a diameter of about 6 µm and are very flexible.

A skilled person is able to select the suitable diameter for the respective application and cell or particle of interest, for example based on the size and rigidity/flexibility of the respective particle or cell of interest. Furthermore, the skilled person knows how to adjust the respective pore size/diameter depending on the surface modifications of the membrane.

According to a preferred embodiment of the present invention, the pore diameter ranges from 5 µm to 12 µm. Further preferred embodiments comprise pore sizes of 5, 6, 7, 8, 9, 10, 11, 12 µm. These pore sizes are particularly preferred for isolating mammalian or human cells that are circulating in the blood stream. For example, by using membranes with such pore diameters it is possible to isolate circulating tumor cells (CTCs) from a blood sample of a cancer patient, because white and red blood cells comprised in a blood sample can pass through pores of this size without getting stuck in the pores, because of their relatively small size and comparably high flexibility.

According to a further preferred embodiment of the present invention, the pores are arranged in an ordered structure on the membrane, wherein the position of the pores is complementary to the position of the wells on the well plate.

The term "ordered structure" refers to an arrangement of the pores and wells in a repetitive and uniform two-dimensional formation on the membrane and on the plate, respectively. For example, the wells or pores of a membrane or plate can be arranged in a way that each pore/well has the same distance to its neighboring pores/wells, leading to a repetitive arrangement of the pores/wells on the membrane/plate.

Furthermore, the pores/wells can be arranged in a rectangular grid, wherein the pores are arranged in horizontal and vertical lines on the membrane and the pores/wells of each line all may have the same distance to its two neighboring pores wells, wherein the horizontal and vertical lines are rectangular to each other. Alternatively, the pores/wells can be arranged in horizontal lines that are shifted against each other, so that the horizontal lines are not rectangular to the vertical lines of pores/wells, but form lines that are diagonal to each other.

Importantly, the wells and pores on the plate and membrane of the present invention can be arranged in corresponding ordered structures, which are complementary to each other. This ensures that upon assembly of the membrane and the plate each pore or the membrane is positioned opposite of the corresponding well of the plate.

According to a preferred embodiment of the present invention, the membrane has thickness of 2-100 µm, preferably 5-20 µm.

It is particularly preferable that the membrane has a thickness of 2-100 µm, because such membranes have the required stability to resist the pressure applied during filtration of the cell suspension, while it is possible to seal the pores of the membrane on top of the corresponding well of the well plate with the cover, for example by mechanical and/or thermic means. A greater thickness allows to apply a higher pressure during filtration or to apply higher mechanical forces, without breaking or rupturing the membrane. Accordingly, thicker membranes are preferable for the filtration of liquids with a high pressure. On the other hand, thin membranes, which are preferably made from thermoplastic materials that allow deformation and fusion with other plastics upon the heating, are preferable to ensure an efficient sealing process with the cover, because already the application of little mechanical forces and/or heat can lead to a deformation of the membrane that allows efficient sealing of the well with the pore and the cells located on top of or in the well.

The thickness of 5-20 µm is particularly preferable because it represents a membrane thickness that ensures stability of the membrane when a filtration pressure is applied that is required for filtering a cell suspension comprising mammalian blood cells and/or circulating tumor cells. On the other hand, a membrane that is 5-20 µm thick is very efficiently sealed and fused with the well plate and the cover, when the cover is applied to the assembled plate and membrane. Furthermore, a membrane with a thickness of 5-20 µm and the comprised pores only add a very small dead volume to the volume of the wells after assembly, which is advantageous for an efficient analysis of the cellular components.

In a particularly preferred embodiment of the method of the present invention, the cells of interest are circulating tumor cells and the immobilized antibodies comprise antibodies with an affinity for EpCAM.

The term "side" in the context of the membrane of the present invention refers to the two flat or plane surfaces of the membrane. The two sides can be designated as a front or supply side and as a back or discharge side. The front side (or supply side) of the membrane is the side that is exposed to the cell suspension during the filtration step. The backside (or discharge side) is the side of the membrane, where the liquid that has passed through the pores of the membrane is released from the pores.

In certain embodiments of the present invention the membrane has two equal sides which are interchangeably with respect to their function as a front or back side during the filtration process. In further preferred embodiments, the membrane can be assembled with the well plate in both directions, either with the front side or the back side facing the well plate.

According to another preferred embodiment of the present invention, the porous membrane has a flat front side, which is exposed to the liquid cell suspension during the filtration process, and the back side comprises means for assembling the porous membrane and the well plate. In this preferred embodiment of the present invention, the back side is positioned on the well plate so that the means for assembling or the porous membrane can engage with the means for assembly of the well plate, which ensures that the pores of the membrane are located opposite of the corresponding well on the membrane.

### Preferred embodiments relating to the filtration and washing of the membrane

Preferred embodiments of the method of the present invention also comprises the provision of means for filtering the liquid cells suspension through the porous membrane.

Means for filtering the liquid cell suspension can be connected to the porous filter membrane in a way that a liquid cell suspension is applied to the front side of the membrane and the flow through of the filtration process may be collected after release from the back side of the membrane. Means for filtering the liquid cell suspension comprise, without limitation, classical filtration systems using filter membranes, such as adapters that can be connected to a membrane and to a syringe, which can be used to apply the liquid cell suspension to the membrane.

Preferably, means for filtering the liquid cells suspension relate to filtration systems that allow the application of a liquid cell suspension to the porous membrane with a controllable filtration pressure (transmembrane pressure). It is particularly preferable that the filtration pressure is controllable, because the filtration pressure can have an influence on the selectivity and specificity of the porous membrane with respect to the cell type that is retained in the pores.

Furthermore, filtration systems of the present invention may comprise hydrostatic systems with liquid reservoirs. Such liquid reservoirs can be advantageous for example to apply buffer solutions to the membrane after filtration for washing the porous membrane and reducing unspecific binding of undesired cells or particles.

The method of the present invention preferably comprises washing of the membrane before assembly with the well plate to remove unspecifically bound cells.

The washing of the membrane after filtration can occur in various automated or manual ways. For example, a filtration buffer or wash buffer can be applied to the membrane from the front side or back side by means of a filtration system with a controlled, specific filtration pressure to remove cells that have loosely bound to the pores of the membrane and can pass through the membrane upon application of additional liquid volume or increased filtration pressure. For example, if the porous membrane has been modified to comprise immobilized antibodies, which are specific for the cells of interest to be isolated, it is possible to remove cells, which got stuck in the pores of the membrane, because they have a similar size as the cells of interest, by increasing the increasing the filtration pressure, while the cells of interest will remain bound to the pores, because they have additionally bound to the antibodies leading to a tighter binding of the cells of interest. Back side washing proved to be particularly efficient to remove contaminating cells or other unspecifically bound material.

In another embodiment of the invention, the membrane can be washed by swinging the membrane in a bowl containing a suitable wash buffer, which will not only remove cells or particles that have unspecifically bound to the pores or the membrane, but also cells or particle that have bound to the plane surfaces of the membrane, specifically to the front side, which has been exposed to the liquid cell suspension during filtration.

### Preferred embodiments of the microscopic analysis and the analysis of cellular components

Preferably, the method of the present invention comprises a microscopic analysis of the at least one pore for retained single cells.

The microscopic analysis step is particularly advantageous in the context of the present invention, because it allows the verification of the identity, phenotype or cell type of the retained single cell that is located in a pore after filtration of a liquid cell suspension through the filter membrane. Accordingly, it is possible to exclude a pore or a well that contains an undesired cell type from the data analysis after performing the method of the present invention and one can be sure for each of the isolated and analyzed single cells, whether it is a cell of interest or a different cell type. Therefore, it is possible to generated data collected from the analysis of multiple single cells of interest that will not be contaminated by results that have been generated due to the analysis of an undesired cell type.

The microscopic analysis can comprise any kind of analysis that is suitable to verify that a cell that is located in a pore of the membrane is a cell of interest, such as, for example, bright field microscopy or immunocytochemistry (ICC) analysis.

Immunocytochemistry (ICC) is a common laboratory technique that is used to visualize the localization of a specific protein or antigen in or on a cell of interest by use of a specific primary antibody that binds to it, for example. The primary antibody allows visualization of the protein under a fluorescence microscope, particularly when it is bound by a secondary antibody that has a conjugated fluorophore.

In the context of the method of the present invention, ICC can be performed using one or more markers of the cell of interest, preferably cell surface markers. In case of the CTCs as cells of interest, suitable markers comprise, without limitation, CK8, CK18, CK19, EpCAM and CD45. Additionally, DNA dyes or nuclear markers, such as DAPI and/or Hoechst, can be used. It is possible to combine several markers in ICC by using distinguishable fluorophores for visualizing the respective marker, for example by means of fluorophore labeled antibodies.

Fluorophores (or fluorochromes) that can be used in the context of the present invention are fluorescent chemical compounds that can re-emit light upon light excitation. Fluorophores for use as labels in constructing labeled biomolecules of the invention comprise, without claiming to be exhaustive, rhodamine and derivatives, such as Texas Red, fluorescein and derivatives, such as 5-bromomethyl fluorescein, Lucifer Yellow, IAEDANS, 7-Me2N-coumarin-4-acetate, 7-OH-4-CH3-coumarin-3-acetate, 7-NH2-4CH3-coumarin-3-acetate (AMCA), monobromobimane, pyrene trisulfonates, such as Cascade Blue, and monobromotrimethyl-ammoniobimane, FAM, TET, CAL Fluor Gold 540, HEX, JOE, VIC, CAL Fluor Orange 560, Cy3, NED, Quasar 570, Oyster 556, TMR, CAL Fluor Red 590, ROX, LC red 610, CAL Fluor Red 610, Texas red, LC red 610, CAL Fluor Red 610, LC red 640, CAL Fluor Red 635, Cy5, LC red 670, Quasar 670, Oyster 645, LC red 705, Cy5.5, BODIPY FL, Oregon Green 488, Rhodamine Green, Oregon Green 514, Cal Gold, BODIPY R6Gj, Yakima Yellow, JOE, HEX, Cal Orange, BODIPY TMR-X, Quasar-570 /Cy3, TAMRA, Rhodamine Red-X, Rhodamine B, Rhodamine 6G, Redmond Red, BODIPY 581/591, Cy3.5, Cal Red/Texas Red, BODIPY TR-X, BODIPY 630/665-X, Pulsar-650, Quasar-670/Cy5.

The microscopic analysis can be performed by means of any suitable microscopy setup and can be carried out manually or in an automated way. It is particularly preferred to perform an automated microscopic analysis of each pore of the porous membrane, because this is time efficient and reduces the work load for the personnel that carry out the analysis. The data generated by microscopic analysis can be mapped to the location or position of each of the isolated single cells on the membrane so that it is possible to link the microscopic data to the position of a single cell on the membrane and/or the well plate and to the data of the analysis of a cellular component. This means that the method of the present invention allows to link the analysis of the cell identity to the analysis of cellular components for each single cell analyzed in the method.

The method of the present invention comprises an analysis of at least one cellular component of a retained single cell in the at least one sealed well.

The term "cellular component" relates to any kind of biomolecule that is comprised by the retained single cell. Biomolecules in the sense of the present invention comprise, but are not limited to, nucleic acids, proteins, enzymes, peptides, antibodies, sugar molecules, lipids and combinations thereof, such as, for example, glycosylated proteins or other glycosylated biomolecules.

Nucleic acids in the context of the present invention comprise, without limitation, DNA, RNA, PNA, ssDNA, dsDNA, RNA, mRNA, tRNA, IncRNA, ncRNA, microRNA, siRNA, rRNA, sgRNA, piRNA, rmRNA, snRNA, snoRNA, scaRNA, gRNA, viral RNA, modified RNA, such as for example LNA. Oligonucleotides in the sense of the present invention are relatively short nucleic acid molecules of about 200 nucleotides in length.

Sugar molecules in the sense of the present invention comprise carbohydrates or saccharides, including mono-, di-, oligo- and polysaccharides. The term glycosylation describes enzymatic and chemical reactions leading to the binding of a sugar molecule to a protein, a lipid or other molecules, wherein the glycosylated product molecules are called glycosides, including glycoproteins, peptidoglycans and glycolipids.

In the context of the present invention, lipids are hydrophobic molecules that are partially or completely insoluble in water and, due to their low polarity, dissolve in lipophilic or hydrophobic solvents. Lipids are important structural components of cell membranes, are used as energy source in living organisms and have functions in signal transduction. Lipids comprise, without limitation, fatty acids, fats, waxes, sterols, fat-soluble vitamins (such as vitamins A, D, E, and K), monoglycerides, diglycerides, triglycerides, phospholipids, sphingolipids, lipopolysaccharides and isoprenoids (terpenoids).

The analysis of the at least one cellular component can relate to, for example, the analysis of the presence, abundance, sequence and/or function of one or more biomolecule of the cell of interest. The analysis of the present invention preferably comprises running of a defined temperature program. Preferred analyses of cellular components of the present invention comprise, without limitation, PCR analysis of one or more nucleic acid sequences, quantitative RT-PCR of one or more genes, whole genome amplification, sequencing of one or more genes, whole genome sequencing, whole transcriptome analysis, in-vitro translation, enzyme assays and electrophoretic analysis.

A preferred embodiment of the present invention relates to a method for analyzing one or more single cells of interest, wherein the analysis of at least one cellular component of a retained cell comprises a nucleic acid amplification.

In a preferred embodiment of the present invention, the cell of interest is a CTC and the analysis of cellular components is directed toward the analysis of expression of tumor suppressor genes and/or oncogenes and/or towards mutation analysis of oncogenes and/or tumor suppressor genes. Further preferred analysis of CTCs relate to the expression of specific markers, which may be analyzed by ICC- or nucleic acid analysis, for example, wherein such markers provide clues about the stem cell properties of the CTC or are relevant for choosing a suitable therapeutic strategy, especially in the context of personalized medicine.

### Preferred embodiments of the well plate and analysis of cellular components

According to preferred embodiments of the present invention, the wells of the plate contain reagents for analyzing at least one component of the cells of interest, wherein the reagents can be provided in liquid or dry form.

In preferred embodiments of the present invention, the reagents are provided in the wells in liquid form. Such embodiments are particularly advantageous, because the reagents do not need to be rehydrated and the liquid reagents are provided in the plate in a ready-to-use format.

The provision of the reagents in dry form is particularly advantageous, if the plates are stored for extended periods before use, because dry reagents may be more stable than reconstituted liquid reagents.

In a preferred embodiment of the method of the present invention, the wells do not contain reagents for analyzing at least one component of the cells of interest. Accordingly, the reagents have to be added to the wells after assembly of the plate and the membrane. This is possible, for example, by means of a microfluidic system, which is connected to the individual wells of the well plate, wherein the microfluidic systems can transport liquid reagents to the individual wells, for example after the well has been sealed.

Preferred embodiments of the present invention comprise means for applying a liquid to the one or more wells of the well plate. The liquid can be, for example, a rehydration buffer or water, which can be used for dissolving or rehydrating dry reagents that are contained in the wells. In further embodiments of the invention, the liquid can comprise the reagents for analyzing the at least one component of the cell of interest, which are applied to the at least one well of the well plate. Means for applying a liquid to one or more wells of the well plate comprise, for example, a pipette, a multichannel pipette or a microfluidic system that is connected to the wells.

Further embodiments of the present invention relate to a method for analyzing one or more single cells of interest, wherein different wells can contain the same or different reagents for analyzing at least one component of the cell of interest.

In a preferred embodiment of the present invention, different wells of the well plate contain the same reagents for analyzing at least one component of the cell of interest. This embodiment is particularly advantageous, because it allows to analyze all isolated single cells in the same way and compare the individual cell with respect to the analyzed component or parameter. The filling of the wells of the plate is simple, because all wells contain identical reagents and there is no risk of contaminating the neighboring wells.

According to another preferred embodiment of the present invention, different wells of the well plate contain different reagents for analyzing at least one component of the cell of interest. For example, in case of a PCR analysis, different wells could contain primers with different sequences that are used for amplifying different nucleic acid sequences. In another embodiment of the present invention, different wells can contain different sequencing barcodes, so that even after pooling the content of different wells after amplification, the amplified nucleic acids can be assigned to a specific cell that was located in a specific pore of the membrane after filtration.

A preferred embodiment of the present invention relates to a method for analyzing one or more single cells of interest, wherein progression of the analysis is detected in real time. For example, the progression of a nucleic acid amplification reaction can be monitored in real time by using intercalating fluorescent dyes, fluorescently labeled nucleic acid probes that change their emission properties during amplification of the target molecule.

Intercalating fluorescent dyes that can be used for real time detection of amplified nucleic acids in the context of the present invention comprise, without limitation, ethidium bromide, propidium iodide, Hoechst 33258, Hoechst 33342, acridine orange, ethidium bromide homodimers, EvaGreen, and all dyes of the SYBR and SYTO families. Such dyes also include POPO, BOBO, YOYO and TOTO from Molecular Probes (Eugene, Oreg.).

It is preferable that the method of the present invention comprises the provision of an adapter for placing the assembled well plate, membrane and cover into an analyzer.

Such adapters of the present invention can be very useful for placing the assembled plate, membrane and cover of the present invention into different analysis machines that are configured to analyze samples that are provided in standard formats. Since the microfabricated plate, membrane and cover of the present invention might not be compatible with commercially available analyzers, suitable adapter can be used. Such adapters are designed in a way that they can be placed into the respective analyzer, while they can also accommodate the assembled well plate, membrane and cover. Preferably, the adapters are thermally conductive so that the can transmit temperature changes of the analyzer to the well plate of the device of the present invention.

By using a variety of different adapters, it may be possible to place the assembled well plate, membrane and cover into many different analyzing machines for different kinds of analytical reactions. Analyzers of the present invention comprise, without limitation, commercially available thermocyclers, thermocyclers for real time detection of amplification reactions, fluorescence readers, plate readers for determining light scattering, nephelometry, fluorescence, luminescence or absorbance, automated microscopes, micromanipulatorsystems, sequencers, or electrophoretic devices.

According to preferred embodiments of the present invention, the at least one well is connected to a microfluidic system that allows transport of liquids from and to the wells.

Microfluidic systems that are connected to the wells of the present invention allow specific transport of liquids from and to the wells of the plate. This enables filling of the wells, for example with reagents for the analysis of cellular components, and transport of the well content, in particular after the analysis reaction (for example a nucleic acid amplification) has occurred, to connected systems that are used for downstream applications.

In preferred embodiments of the present invention, the at least one component of the retained single cell is used for downstream processing after analysis. Downstream applications or analysis of the analyzed components of the single cells of interest may be, for example, sequencing of analysis of amplified nucleic acid sequences, whole genome sequencing after whole genome amplification or electrophoretic analysis of amplified nucleic acid sequences.

The transport of the analyzed cellular components from the wells to the devices or systems for downstream applications can occur via microfluidic systems and/or complex adapter systems. Such complex adapter systems can comprise microfluidic systems that are connected to the microfluidic system of the well plate and/or of the systems for downstream analysis of the analyzed cellular components.

In alternative embodiments of the present invention, the analyzed cellular components, such as amplified nucleic acid molecules, could be removed from the wells by means of a micromanipulator, which might penetrate through the cover and/or the membrane on top of the well, wherein the cover and/or the membrane can have a predetermined breaking point, where the micromanipulator could penetrate through the cover and/or the membrane. In preferred embodiments of the invention, this step of removing the analyzed cellular components from the wells can be performed by a micromanipulator that comprises multiple parallel needles that can penetrate multiple wells in parallel for removing the liquid comprising the reaction products.

The shape of the at least one well of the well plate can be freely chosen. Wells of the present invention can have any shape that is compatible with the features of the present invention, including, without limitation, cylindrical, conical, flat bottom or round bottom wells. Conical or cylindrical wells may be preferred, because the enable a reduction of the volume of the wells while still having a wide diameter at the opening or top of the well, which surrounds the pore comprising the cell of interest after assembly of the plate, the membrane and the cover.

### Further preferred embodiments of the present invention

The membrane and or the plate of the present invention comprise means for assembling the porous membrane and the well plate in an orientation to position the at least one pore opposite of the at least one well. Such means for assembling are particularly important to ensure that each pore of the membrane is positioned opposite of the corresponding well of the plate after assembly. Suitable means in the sense of the present invention comprise mechanical structures that ensure that the membrane and the plate can only be assembled in a specific way that ensures the correct orientation of the wells and the pores. Furthermore, optical marks or labels might be used that indicate how the membrane and the plate should be oriented to each other to ensure the optimal positioning of the pores and the wells. Such marks may also be holes in the membrane, that can either serve as optical marks or may engage with corresponding structures of the well plate or cover.

A further preferred embodiment relates to the method for analyzing one or more single cells of interest, wherein the means for assembling are complementary mechanical structures of the membrane and the well plate, wherein the mechanical structures of membrane and the mechanical structures of the well plate engage with each other when the membrane and the well plate are assembled in an orientation to position the at least one pore opposite of the at least one well and prevent assembly of the membrane and the well plate in a different orientation.

The present invention comprises a cover for sealing the at least one well with the at least one pore localized opposite of the at least one well after assembling the porous membrane and the well plate. The cover can be combined with the membrane before or after assembling the membrane and the well plate. The cover should be designed in a way that it can tightly seal the wells, while the membrane with the pores and the retained cell of interest are locked in between the cover and the edges of the respective well.

This can be achieved, for example, by mechanically and/or thermally by pressing the cover onto the assembled membrane and plate, preferably while heat is applied, which is a process that is similar to classical lamination. Furthermore, the sealing process can involve additional chemical processes, such as reactive lamination processes, which are based on polymer coatings that can be thermally crosslinkable and/or photocrosslinkable. Preferably, the sealing occurs through a mechanical-thermal reaction or a chemical reaction.

In preferred embodiments of the present invention, the cover plate and/or the well plate and/or the membrane of the present invention are coated with a reactive lamination polymer, which enables crosslinking of the polymer, for example upon thermal or photo stimulation. Such methods are known to the person skilled in the art and have been described in the literature, as for example by Kelb et al. (Kelb, C.; Rother, R.; Schuler, A.-K.; Hinkelmann, M.; Rahlves, M. et al.: Manufacturing of embedded multimode waveguides by reactive lamination of cyclic olefin polymer and polymethylmethacrylate. Optical Engineering 55 (2016), Nr. 3, 37103. DOI: https://doi.org/10.1117/1.OE.55.3.037103).

According to further preferred embodiments, the sealing process can be based on or involve additionally the insertion into oil.

The present invention also relates to a device for analyzing one or more single cells of interest, comprising
- a porous membrane comprising at least one pore, wherein the pore is adjusted to trap a single cell of interest when the cell is applied to the membrane in a liquid medium and to allow smaller or more flexible cells of a cell suspension to pass through the pore
- a well plate comprising at least one well, wherein the well diameter is larger than the pore diameter and the at least one well contains reagents for analyzing at least one component of the cells of interest, wherein the reagents can be provided in liquid or dry form,
- means for assembling the porous membrane and the well plate in an orientation to position the at least one pore opposite of the at least one well, wherein the means for assembling are part of the porous membrane and/or the well plate,
- a cover for sealing the at least one well with the at least one pore localized opposite of the at least one well after assembling the porous membrane and the well plate.

Preferably, all components of a device of the present invention are transparent and allow the transmission of light.

In embodiments of the device of the invention, in the assembled configuration, a well of the well plate can only be in contact with one pore of the porous membrane. Accordingly, after performing the method of the invention using the device of the invention a single well is usually only in contact with a single cell that has been retained in a pore of the porous membrane. This is a great advantage of the device of the invention in comparison to filter plate systems of the state of the art that comprise strainer wells with multiple pores in a mesh, wherein the strainer well is located opposite of a well of reservoir plate, so that multiple pores that may have retained multiple cells are located in a single well. Accordingly, analysis of an individual retained cells in the well of the reservoir is not possible, in contrast to the system of the invention. The invention further relates to a system for analyzing one or more single cells of interest, comprising
- a device for analyzing one or more single cells of interest according to the present invention, and
- means for filtering a liquid cell suspension comprising cells of interest through the porous membrane, and/or
- a microscopy setup for analyzing the at least one pore of the membrane for retained single cells, and/or
- an analyzer for analysis of at least one cellular component of a retained single cell in the at least one sealed well.

Additionally, the present invention relates to a kit for analyzing one or more single cells of interest, comprising
- a device for analyzing one or more single cells of interest according to the present invention, and
- reagents for visualizing retained cells on the porous membrane for microscopic analysis, and/or
- reagents for analysis of at least one cellular component of a retained single cell in the at least one sealed well.

The person skilled in the art knows that the features, properties and advantages recited above for the method for analyzing one or more single cells of interest of the present invention equally apply to the device, system and kit for analyzing one or more single cells of interest of the present invention. It is obvious that the preferred features of the method of the invention can be integrated into device, system or kit, since the method is performed with help of the device, system or kit. Accordingly, all features and advantages of the method of the present invention have also been disclosed in the context of the device, system or kit. The other way around, features that have been disclosed in the context of the device, system or kit also apply to the method of the present invention.

### Detailed Description of the invention

The invention is further described by the following figures. These are not intended to limit the scope of the invention, but represent preferred embodiments of aspects of the invention provided for greater illustration of the invention described herein.

### Brief description of the figures:

*Figure 1**:* Schematic of the proposed platform for the enrichment and single-cell analysis of CTCs.
*Figure 2**:* Schematic of a modular product enabling single-cell studies on captured CTCs using standard laboratory equipment.

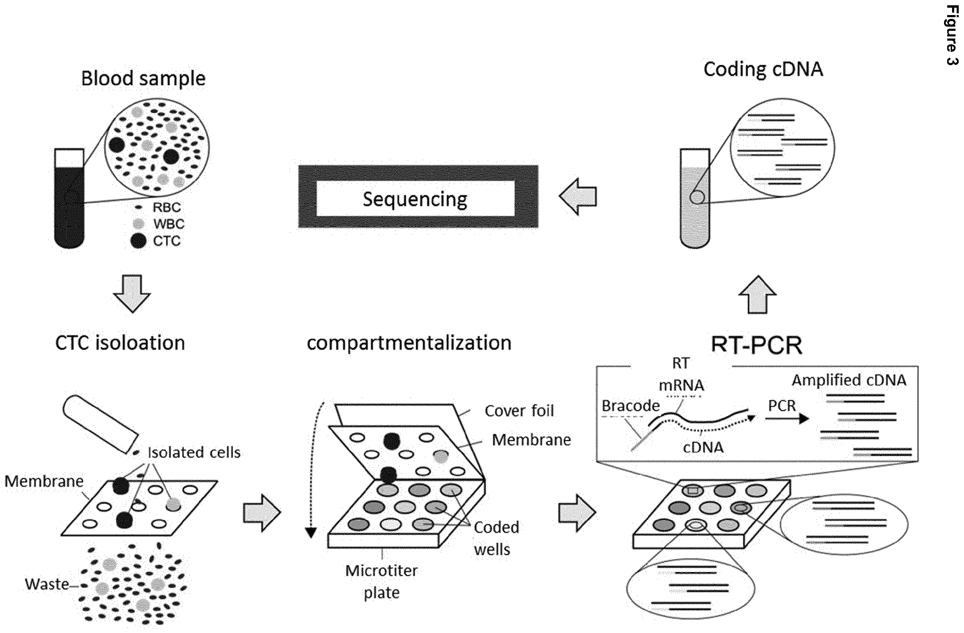

*Figure 3**:* Schematic representation of a proposed workflow for the efficient coupling of isolation and sequencing of individual circulating tumor cells.

### Examples

The invention is further described by the following examples. These are not intended to limit the scope of the invention, but represent preferred embodiments of aspects of the invention provided for greater illustration of the invention described herein.

### Example 1:

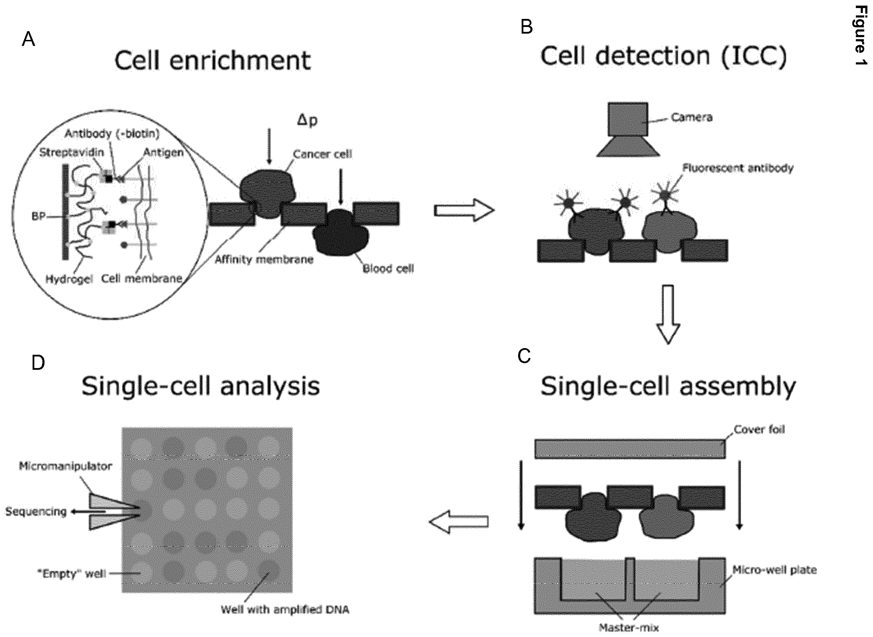

The following is a description of the integrated analysis platform described by the drawings of Figure 1.

At the heart of the platform lies a membrane, which enables the enrichment of cells of interest, such as cancer cells or CTCs, using physical separation criteria. Optionally, the membrane can also use affinity methods for retention and isolation of cells of interest, for example antibodies. This combined approach, which has recently been introduced (31) can improve the capture performance of classical micro-filtration and enables high quality cell analysis (Figure 1 A). To allow for subsequent single-cell analysis, a membrane with precisely defined pore locations is used. After cell enrichment such a membrane contains the captured CTCs well separated and at well-defined locations (i.e. in/on the pores). This means that the two initial steps, which are required for the single-cell analysis of CTCs (i.e. the enrichment and the separation of single-cells), are accomplished at once. The captured cells are then observed and identified using standard microscopy and ICC protocols (Figure 1 B).

To avoid further cell transfer steps to enable molecular single-cell analysis, an innovative assembly is used that transforms each pore into an individual reaction chamber by sandwiching the membrane between a cover foil and a tailor-made micro-well plate containing the necessary components (i.e. the master-mix) (Figure 1C). The tightly sealed detection assembly is subsequently inserted into a standard thermocycler to perform the desired amplification reactions (i.e. PCR or RT-PCR for targeted amplification or WGA or WTA for whole-genome or whole-transcriptome amplification). In the case of a targeted amplification, fluorescent imaging of the detection assembly during or after (RT-)PCR immediately yields molecular information from each single cell and enables direct correlation with its morphology or ICC data captured earlier.

Due to the high pore density of the membrane, ten thousands of single-cell analysis can easily be performed on an area as small as 1 cm² enabling high throughputs. In the case of WGA or WTA, wells containing successfully amplified material can be identified by fluorescence imaging and selected for subsequent sequencing. The content of the selected wells could be retrieved using a micromanipulator and used as starting material for standard library preparation for NGS (Figure 1D). Importantly, the handling of the amplified material during this step is considered less critical compared to the one of single-cells or their non-amplified DNA or RNA during earlier stages of conventional workflows.

### Example 2:

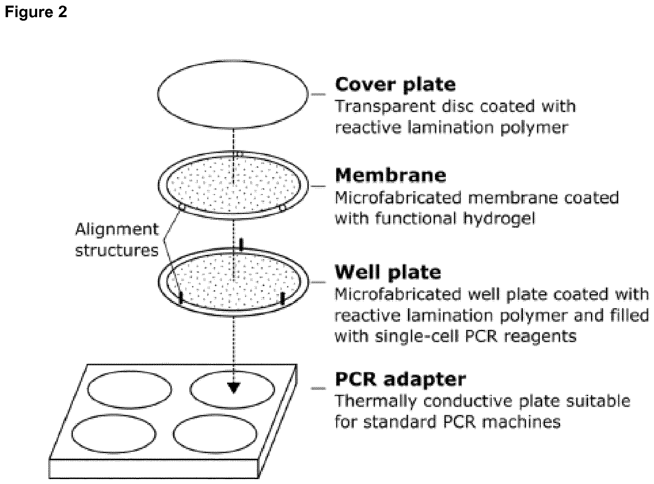

To provide high application flexibility and minimize costs to the end-user (e.g. hospitals, pharmaceutical companies, service labs and universities) a modular product design is proposed that is compatible with existing microscope setups and PCR machines (Figure 2).

The modular product comprises a cover plate, which is a transparent disc coated with a reactive lamination polymer; a porous microfabricated membrane, which is coated with functional hydrogel; a microfabricated well plate, which is coated with a reactive lamination polymer and filled with single-cell PCR reagents; a PCR adapter, which is a thermally conductive plate suitable for standard PCR machines. The well plate and the membrane comprise complimentary means for assembling (alignment structures).

### Example 3:

The proposed exemplary workflow comprises the isolation of CTC from a blood sample by filtering the sample through a membrane consisting of a thin plate, which has cylindrical pores of exact pore diameter at precisely defined positions, and which may be provided with a functional hydrogel coating. This membrane allows the isolation of CTCs from whole blood via size selection and/or molecular interaction with the functional coating (i.e. via surface proteins expressed by the cells). After filtration, the isolated cells can be analyzed by conventional microscopic methods on the membrane. In order to enable the single-cell sequencing of the isolated cells in the most efficient way, the membrane is now superimposed with the trapped cells with a microtiter plate. As a result, each pore is welded to exactly one underlying well on the microtiter plate. Since the individual wells have previously been provided with the necessary reagents for a reverse transcriptase polymerase chain reaction (RT-PCR) and individual barcodes, it is possible to generated cDNAfrom cell-specific mRNA molecules in all the wells, which contain individual cells after assembly with membrane. The products from all pots can then be pooled and analyzed with a commercial sequencer. Due to the built-in barcodes, the read out sequences can be assigned to the corresponding cells. Such a platform allows a broad user base to efficiently sequence CTCs from patient samples using existing devices and sequencing them without transfer steps.

### References

1. Stewart, B. W. & Wild, C. P. World cancer report 2014. World Health Organization (2014).doi:9283204298
2. Ferlay, J. et al. Cancer incidence and mortality patterns in Europe: Estimates for 40 countries in 2012. Eur. J. Cancer 49, 1374-1403 (2013).
3. Eccles, S. A. & Welch, D. R. Metastasis: recent discoveries and novel treatment strategies. Lancet 369, 1742-1757 (2007).
4. Pantel, K., Brakenhoff, R. H. & Brandt, B. Detection, clinical relevance and specific biological properties of disseminating tumour cells. Nat. Rev. Cancer 8, 329-340 (2008).
5. Krebs, M. G. et al. Molecular analysis of circulating tumour cells-biology and biomarkers. Nat. Rev. Clin. Oncol. 11, 129-144 (2014).
6. Alix-Panabier es, C. & Pantel, K. Circulating tumor cells: Liquid biopsy of cancer. Clin. Chem. 59, 110-118 (2013).
7. Joosse, S. A., Gorges, T. M. & Pantel, K. Biology , detection , and clinical implications ofcirculating tumor cells. EMBO Mol. Med. 7, 1-11 (2015).
8. Alix-Panabieres, C. & Pantel, K. Challenges in circulating tumour cell research. Nat. Rev. Cancer 14, 623-631 (2014).
9. Zhang, C., Guan, Y., Sun, Y., Ai, D. & Guo, Q. Tumor heterogeneity and circulating tumor cells. Cancer Lett. 374, 216-223 (2016).
10. Van Loo, P. & Voet, T. Single cell analysis of cancer genomes. Curr. Opin. Genet. Dev. 24, 82-91 (2014).
11. Quin, F. W. & Mead, A. J. Application of single-cell genomics in cancer: Promise and challenges. Hum. Mol. Genet. 24, R74-R84 (2015).
12. Ren, S. C., Qu, M. & Sun, Y. H. Investigating intratumour heterogeneity by single-cell sequencing. Asian J Androl 15, 729-734 (2013).
13. Khoo, B. L., Chaudhuri, P. K., Ramalingam, N., Shao, D. & Tan, W. Single-cell profiling heterogeneity approaches to probing tumor heterogeneity. Int. J. Cancer 0, 1-13 (2016).
14. Pantel, K. & Speicher, M. R. The biology of circulating tumor cells. Oncogene 35,1216-1224 (2016).
15. Heath, J. R., Ribas, A. & Mischel, P. S. Single-cell analysis tools for drug discovery anddevelopment. Nat. Rev. Drug Discov. 15, 204-216 (2016).
16. Patriarca, C., Macchi, R. M., Marschner, A. K. & Mellstedt, H. Epithelial cell adhesion molecule expression (CD326) in cancer: a short review. Cancer Treat. Rev. 38, 68-75 (2012).
17. Liu, H. et al. The biological and clinical importance of epithelial-mesenchymal transition in circulating tumor cells. J. Cancer Res. Clin. Oncol. 189-201 (2014). doi:10.1007/s00432-014-1752-x
18. Cima, I. et al. Label-free isolation of circulating tumor cells in microfluidic devices: Currentresearch and perspectives. Biomicrofluidics 7, 11810 (2013).
19. Jin, C. et al. Technologies for label-free separation of circulating tumor cells: from historical foundations to recent developments. Lab Chip 14, 32-44 (2014).
20. Kim, M. S. et al. SSA-MOA: a novel CTC isolation platform using selective size amplification (SSA) and a multi-obstacle architecture (MOA) filter. Lab Chip 12, 2874-2880 (2012).
21. Macaulay, I. C. & Voet, T. Single Cell Genomics: Advances and Future Perspectives. PLoS Genet. 10, e1004126 (2014).
22. Thompson, A. M., Paguirigan, A. L., Kreutz, J. E., Radich, J. P. & Chiu, D. T. Microfluidics for single-cell genetic analysis. Lab Chip 14, 3135-3142 (2014).
23. Navin, N. E. Cancer genomics: one cell at a time. Genome Biol. 15, 452 (2014).
24. Navin, N. E. The first five years of single-cell cancer genomics and beyond. Genome Res. 25, 1499-1507 (2015).
25. Wang, Y. & Navin, N. E. Advances and Applications of Single-Cell Sequencing Technologies. Mol. Cell 58, 598-609 (2015).
26. Gawad, C., Koh, W. & Quake, S. R. Single-cell genome sequencing: current state of the science. Nat. Rev. Genet. 17, 175-188 (2016).
27. Zhang, X. et al. Single-Cell sequencing for precise cancer research: Progress and prospects. Cancer Res. 76, 1305-1312 (2016).
28. Reece, A. et al. Microfluidic techniques for high throughput single cell analysis. Curr. Opin. Biotechnol. 40, 90-96 (2016).
29. Konry, T., Sarkar, S., Sabhachandani, P. & Cohen, N. Innovative Tools and Technology for Analysis of Single Cells and Cell-Cell Interaction. Annu. Rev. Biomed. Eng. 18, 259-84 (2016).
30. Lohr, J. G. et al. Whole-exome sequencing of circulating tumor cells provides a window into metastatic prostate cancer. Nat. Biotechnol. 32, 479-484 (2014).
31. Zinggeler, M., Brandstetter, T. & Rühe, J. Towards high performance detection of circulating tumor cells in whole blood. Procedia Eng. 120, 380-383 (2015).

## Claims

1. Method for analyzing one or more single cells of interest, comprising
- provision of
i. a porous membrane comprising at least one pore, wherein the pore is adjusted to trap a single cell of interest when the cell is applied to the membrane in a liquid medium and to allow smaller or more flexible cells of the cell suspension to pass through the pore,
ii. a well plate comprising at least one well, wherein the well diameter is larger than the pore diameter and the at least one well contains reagents for analyzing at least one component of the cells of interest, wherein the reagents can be provided in liquid or dry form,
iii. means for assembling the porous membrane and the well plate in an orientation to position the at least one pore opposite of the at least one well, wherein the means for assembling are part of the porous membrane and/or the well plate,
iv. a cover for sealing the at least one well with the at least one pore localized opposite of the at least one well after assembling the porous membrane and the well plate, and
- filtering a liquid cell suspension comprising cells of interest through the porous membrane,
- assembling the porous membrane and the well plate in an orientation to position the at least one pore opposite of the at least one well,
- sealing the at least one well and the at least one pore localized opposite of the well with the cover,
- analysis of at least one cellular component of a retained single cell in the at least one sealed well.

2. The method for analyzing one or more single cells of interest according to claim 1, wherein the membrane is coated with a hydrogel and/or comprises immobilized antibodies.

3. The method for analyzing one or more single cells of interest according to any one of the preceding claims, wherein the method additionally comprises a microscopic analysis of the at least one pore for retained single cells.

4. The method for analyzing one or more single cells of interest according to any one of the preceding claims, wherein the membrane has thickness of 2-100 µm, preferably 5-20 µm.

5. The method for analyzing one or more single cells of interest according to any one of the preceding claims, wherein the pores are arranged in an ordered structure on the membrane, wherein the position of the pores is complementary to the position of the wells on the well plate.

6. The method for analyzing one or more single cells of interest according to any one of the preceding claims, wherein the porous membrane has a flat front side, which is exposed to the liquid cell suspension during the filtering process, and the back side comprises the means for assembling the porous membrane and the well plate.

7. The method for analyzing one or more single cells of interest according to any one of the preceding claims, wherein the pore diameter ranges from 5 µm to 12 µm.

8. The method for analyzing one or more single cells of interest according to any one of the preceding claims, wherein different wells can contain the same or different reagents for analyzing at least one component of the cell of interest.

9. The method for analyzing one or more single cells of interest according to one of the preceding claims, wherein the at least one well is connected to a microfluidic system that allows transport of liquids from and to the wells.

10. The method for analyzing one or more single cells of interest according to any one of the preceding claims, wherein the cells of interest are circulating tumor cells and the immobilized antibodies comprise antibodies with an affinity for EpCAM.

11. The method for analyzing one or more single cells of interest according to any one of the preceding claims, wherein the analysis of at least one cellular component of a retained cell comprises a nucleic acid amplification, and/or wherein progression of the analysis is detected in real time.

12. The method for analyzing one or more single cells of interest according to any one of the preceding claims, wherein the sealing occurs through a mechanical-thermal reaction or a chemical reaction, and/or wherein the at least one component of the retained single cell is used for downstream processing after analysis.

13. Device for analyzing one or more single cells of interest, comprising
- a porous membrane comprising at least one pore, wherein the pore is adjusted to trap a single cell of interest when the cell is applied to the membrane in a liquid medium and to allow smaller or more flexible cells of the cell suspension to pass through the pore,
- a well plate comprising at least one well, wherein the well diameter is larger than the pore diameter,
- means for assembling the porous membrane and the well plate in an orientation to position the at least one pore opposite of the at least one well, wherein the means for assembling are part of the porous membrane and/or the well plate,
- a cover for sealing the at least one well with the at least one pore localized opposite of the at least one well after assembling the porous membrane and the well plate.

14. System for analyzing one or more single cells of interest, comprising
- a device for analyzing one or more single cells of interest according to claim 13, and
- means for filtering a liquid cell suspension comprising cells of interest through the porous membrane, and/or
- a microscopy setup for analyzing the at least one pore of the membrane for retained single cells, and/or
- an analyzer for analysis of at least one cellular component of a retained single cell in the at least one sealed well.

15. Kit for analyzing one or more single cells of interest, comprising
- a device for analyzing one or more single cells of interest according to claim 13, and
- reagents for visualizing retained cells on the porous membrane for microscopic analysis, and/or
- reagents for analysis of at least one cellular component of a retained single cell in the at least one sealed well.

## Patentansprüche

1. Verfahren zur Analyse von einer oder mehreren interessierenden Einzelzellen, umfassend:
- Bereitstellung von
i. einer porösen Membran, die mindestens eine Pore umfasst, wobei die Pore eingestellt ist, um eine interessierende Einzelzelle einzuschließen, wenn die Zelle in einem flüssigen Medium auf die Membran aufgebracht wird, und um kleineren oder flexibleren Zellen der Zellsuspension zu ermöglichen, durch die Pore zu gehen,
ii. eine Mikrotiterplatte, die mindestens eine Vertiefung umfasst, wobei der Vertiefungsdurchmesser größer als der Porendurchmesser ist und die mindestens eine Vertiefung Reagenzien zur Analyse mindestens eines Bestandteils der interessierenden Zellen enthält, wobei die Reagenzien in flüssiger oder trockener Form bereitgestellt werden können,
iii. Mittel zur Montage der porösen Membran und der Mikrotiterplatte in einer Ausrichtung, um die mindestens eine Pore gegenüber der mindestens einen Vertiefung zu positionieren, wobei die Mittel zur Montage Teil der porösen Membran und/oder der Mikrotiterplatte sind,
iv. eine Abdeckung zur Abdichtung der mindestens einen Vertiefung mit der mindestens einen Pore, die sich nach der Montage der porösen Membran und der Mikrotiterplatte gegenüber der mindestens einen Vertiefung befindet, und
- Filterung einer flüssigen Zellsuspension, die interessierende Zellen umfasst, durch die poröse Membran,
- Montage der porösen Membran und der Mikrotiterplatte in einer Ausrichtung, um die mindestens eine Pore gegenüber der mindestens einen Vertiefung zu positionieren,
- Abdichtung der mindestens einen Vertiefung und der mindestens einen Pore, die sich gegenüber der Vertiefung befindet, mit der Abdeckung,
- Analyse des mindestens einen zellulären Bestandteils einer zurückgehaltenen Einzelzelle in der mindestens einen abgedichteten Vertiefung.

2. Verfahren zur Analyse einer oder mehrerer interessierender Einzelzellen nach Anspruch 1, wobei die Membran mit einem Hydrogel beschichtet ist und/oder immobilisierte Antikörper umfasst.

3. Verfahren zur Analyse einer oder mehrerer interessierender Einzelzellen nach einem der vorhergehenden Ansprüche, wobei das Verfahren zusätzlich eine mikroskopische Analyse der mindestens einen Pore für zurückgehaltene Einzelzellen umfasst.

4. Verfahren zur Analyse einer oder mehrerer interessierenden Einzelzellen nach einem der vorhergehenden Ansprüche, wobei die Membran eine Dicke von 2-100 µm, bevorzugt 5-20 µm, aufweist.

5. Verfahren zur Analyse einer oder mehrerer interessierender Einzelzellen nach einem der vorhergehenden Ansprüche, wobei die Poren in einer geordneten Struktur auf der Membran angeordnet sind, wobei die Position der Poren eine Ergänzung zu der Position der Vertiefungen auf der Mikrotiterplatte ist.

6. Verfahren zur Analyse einer oder mehrerer interessierender Einzelzellen nach einem der vorhergehenden Ansprüche, wobei die poröse Membran eine flache Vorderseite aufweist, welche der flüssigen Zellsuspension während des Filterprozesses exponiert ist, und wobei die Rückseite die Mittel zur Montage der porösen Membran und der Mikrotiterplatte umfasst.

7. Verfahren zur Analyse einer oder mehrerer interessierender Einzelzellen nach einem der vorhergehenden Ansprüche, wobei der Porendurchmesser von 5 µm bis 12 µm reicht.

8. Verfahren zur Analyse einer oder mehrerer interessierender Einzelzellen nach einem der vorhergehenden Ansprüche, wobei unterschiedliche Vertiefungen dieselben oder unterschiedliche Reagenzien zur Analyse von mindestens einem Bestandteil der interessierenden Zelle enthalten können.

9. Verfahren zur Analyse einer oder mehrerer interessierender Einzelzellen nach einem der vorhergehenden Ansprüche, wobei die mindestens eine Vertiefung mit einem mikrofluidischen System verbunden ist, das die Beförderung von Flüssigkeiten von und zu den Vertiefungen ermöglicht.

10. Verfahren zur Analyse einer oder mehrerer interessierender Einzelzellen nach einem der vorhergehenden Ansprüche, wobei die interessierenden Zellen zirkulierende Tumorzellen sind und die immobilisierten Antikörper Antikörper mit einer Affinität für EpCAM umfassen.

11. Verfahren zur Analyse einer oder mehrerer interessierender Einzelzellen nach einem der vorhergehenden Ansprüche, wobei die Analyse von mindestens einem zellulären Bestandteil einer zurückgehaltenen Zelle eine Nukleinsäureamplifikation umfasst, und/oder wobei der Verlauf der Analyse in Echtzeit ermittelt wird.

12. Verfahren zur Analyse einer oder mehrerer interessierender Einzelzellen nach einem der vorhergehenden Ansprüche, wobei die Abdichtung durch eine mechanischthermische Reaktion oder eine chemische Reaktion eintritt, und/oder wobei der mindestens eine Bestandteil der zurückgehaltenen Einzelzelle zur stromabwärtigen Verarbeitung nach der Analyse verwendet wird.

13. Vorrichtung zur Analyse einer oder mehrerer interessierender Einzelzellen, umfassend:
- eine poröse Membran, die mindestens eine Pore umfasst, wobei die Pore eingestellt ist, um eine interessierende Einzelzelle einzuschließen, wenn die Zelle in einem flüssigen Medium auf die Membran aufgebracht wird, und um kleineren oder flexibleren Zellen der Zellsuspension zu ermöglichen, durch die Pore zu gehen,
- eine Mikrotiterplatte, die mindestens eine Vertiefung umfasst, wobei der Vertiefungsdurchmesser größer als der Porendurchmesser ist,
- Mittel zur Montage der porösen Membran und der Mikrotiterplatte in einer Ausrichtung, um die mindestens eine Pore gegenüber der mindestens einen Vertiefung zu positionieren, wobei die Mittel zur Montage Teil der porösen Membran und/oder der Mikrotiterplatte sind,
- eine Abdeckung zur Abdichtung der mindestens einen Vertiefung mit der mindestens einen Pore, die sich nach der Montage der porösen Membran und der Mikrotiterplatte gegenüber der mindestens einen Vertiefung befindet.

14. System zur Analyse einer oder mehrerer interessierender Einzelzellen, umfassend:
- eine Vorrichtung zur Analyse einer oder mehrerer interessierender Einzelzellen nach Anspruch 13, und
- Mittel zur Filterung einer flüssigen Zellsuspension, die interessierende Zellen umfasst, durch die poröse Membran, und/oder
- einen Mikroskopieaufbau zur Analyse der mindestens einen Pore der Membran für zurückgehaltene Einzelzellen, und/oder
- ein Analysegerät zur Analyse von mindestens einem zellulären Bestandteil einer zurückgehaltenen Einzelzelle in der mindestens einen abgedichteten Vertiefung.

15. Kit zur Analyse einer oder mehrerer interessierender Einzelzellen, umfassend:
- eine Vorrichtung zur Analyse einer oder mehrerer interessierender Einzelzellen nach Anspruch 13, und
- Reagenzien zur Visualisierung zurückgehaltener Zellen auf der porösen Membran zur mikroskopischen Analyse, und/oder
- Reagenzien zur Analyse von mindestens einem zellulären Bestandteil einer zurückgehaltenen Einzelzelle in der mindestens einen abgedichteten Vertiefung.

## Revendications

1. Procédé pour analyser une ou plusieurs cellules uniques d'intérêt, comprenant
- la fourniture de
i. une membrane poreuse comprenant au moins un pore, dans lequel le pore est ajusté pour piéger une cellule unique d'intérêt lorsque la cellule est appliquée sur la membrane dans un milieu liquide et pour permettre aux cellules plus petites ou plus souples de la suspension cellulaire de passer à travers le pore,
ii. une plaque à puits comprenant au moins un puits, dans lequel le diamètre de puits est plus grand que le diamètre de pore et l'au moins un puits contient des réactifs pour analyser au moins un composant des cellules d'intérêt, dans lequel les réactifs peuvent être fournis sous forme liquide ou sèche,
iii. un moyen d'assemblage de la membrane poreuse et de la plaque à puits dans une orientation pour positionner l'au moins un pore à l'opposé de l'au moins un puits, dans lequel le moyen d'assemblage fait partie de la membrane poreuse et/ou de la plaque à puits,
iv. un couvercle pour sceller l'au moins un puits avec l'au moins un pore localisé à l'opposé de l'au moins un puits après assemblage de la membrane poreuse et de la plaque à puits, et
- la filtration d'une suspension cellulaire liquide comprenant des cellules d'intérêt à travers la membrane poreuse,
- l'assemblage de la membrane poreuse et de la plaque à puits dans une orientation pour positionner l'au moins un pore à l'opposé de l'au moins un puits,
- le scellement de l'au moins un puits et de l'au moins un pore localisé à l'opposé du puits avec le couvercle,
- l'analyse d'au moins un composant cellulaire d'une cellule unique retenue dans l'au moins un puits scellé.

2. Procédé pour analyser une ou plusieurs cellules uniques d'intérêt selon la revendication 1, dans lequel la membrane est revêtue avec un hydrogel et/ou comprend des anticorps immobilisés.

3. Procédé pour analyser une ou plusieurs cellules uniques d'intérêt selon l'une quelconque des revendications précédentes, dans lequel le procédé comprend en outre une analyse microscopique de l'au moins un pore pour déceler des cellules uniques retenues.

4. Procédé pour analyser une ou plusieurs cellules uniques d'intérêt selon l'une quelconque des revendications précédentes, dans lequel la membrane a une épaisseur de 2 à 100 µm, de préférence de 5 à 20 µm.

5. Procédé pour analyser une ou plusieurs cellules uniques d'intérêt selon l'une quelconque des revendications précédentes, dans lequel les pores sont agencés dans une structure ordonnée sur la membrane, dans lequel la position des pores est complémentaire avec la position des puits sur la plaque à puits.

6. Procédé pour analyser une ou plusieurs cellules uniques d'intérêt selon l'une quelconque des revendications précédentes, dans lequel la membrane poreuse a une face avant plane, qui est exposée à la suspension cellulaire liquide pendant le procédé de filtration, et la face arrière comprend le moyen d'assemblage de la membrane poreuse et de la plaque à puits.

7. Procédé pour analyser une ou plusieurs cellules uniques d'intérêt selon l'une quelconque des revendications précédentes, dans lequel le diamètre de pore se situe dans une plage de 5 µm à 12 µm.

8. Procédé pour analyser une ou plusieurs cellules uniques d'intérêt selon l'une quelconque des revendications précédentes, dans lequel différents puits peuvent contenir des réactifs identiques ou différents pour analyser au moins un composant de la cellule d'intérêt.

9. Procédé pour analyser une ou plusieurs cellules uniques d'intérêt selon l'une des revendications précédentes, dans lequel l'au moins un puits est relié à un système microfluidique qui permet le transport de liquides depuis et vers les puits.

10. Procédé pour analyser une ou plusieurs cellules uniques d'intérêt selon l'une quelconque des revendications précédentes, dans lequel les cellules d'intérêt sont des cellules tumorales circulantes et les anticorps immobilisés comprennent des anticorps ayant une affinité pour l'EpCAM.

11. Procédé pour analyser une ou plusieurs cellules uniques d'intérêt selon l'une quelconque des revendications précédentes, dans lequel l'analyse d'au moins un composant cellulaire d'une cellule retenue comprend une amplification d'acide nucléique, et/ou dans lequel la progression de l'analyse est détectée en temps réel.

12. Procédé pour analyser une ou plusieurs cellules uniques d'intérêt selon l'une quelconque des revendications précédentes, dans lequel le scellement se produit au travers d'une réaction mécanique-thermique ou d'une réaction chimique, et/ou dans lequel l'au moins un composant de la cellule retenue unique est utilisé pour un traitement en aval après analyse.

13. Dispositif pour analyser une ou plusieurs cellules uniques d'intérêt, comprenant
- une membrane poreuse comprenant au moins un pore, dans lequel le pore est ajusté pour piéger une cellule unique d'intérêt lorsque la cellule est appliquée sur la membrane dans un milieu liquide et pour permettre aux cellules plus petites ou plus souples de la suspension cellulaire de passer à travers le pore,
- une plaque à puits comprenant au moins un puits, dans lequel le diamètre de puits est plus grand que le diamètre de pore,
- un moyen d'assemblage de la membrane poreuse et de la plaque à puits dans une orientation pour positionner l'au moins un pore à l'opposé de l'au moins un puits, dans lequel le moyen d'assemblage fait partie de la membrane poreuse et/ou de la plaque à puits,
- un couvercle pour sceller l'au moins un puits avec l'au moins un pore localisé à l'opposé de l'au moins un puits après assemblage de la membrane poreuse et de la plaque à puits.

14. Système pour analyser une ou plusieurs cellules uniques d'intérêt, comprenant
- un dispositif pour analyser une ou plusieurs cellules uniques d'intérêt selon la revendication 13, et
- un moyen de filtration d'une suspension cellulaire liquide comprenant des cellules d'intérêt à travers la membrane poreuse, et/ou
- une installation de microscopie pour analyser l'au moins un pore de la membrane pour déceler des cellules uniques retenues, et/ou
- un analyseur pour l'analyse d'au moins un composant cellulaire d'une cellule unique retenue dans l'au moins un puits scellé.

15. Kit pour analyser une ou plusieurs cellules uniques d'intérêt, comprenant
- un dispositif pour analyser une ou plusieurs cellules uniques d'intérêt selon la revendication 13, et
- des réactifs pour visualiser des cellules retenues sur la membrane poreuse pour l'analyse microscopique, et/ou
- des réactifs pour l'analyse d'au moins un composant cellulaire d'une cellule unique retenue dans l'au moins un puits scellé.
